# FASCICULE DE BREVET EUROPEEN

(11) **EP 3 331 637 B1**
(45) Date de publication et mention de la délivrance du brevet: **22.01.2025**
(21) Numéro de dépôt: 16757705.5
(22) Date de dépôt: 02.08.2016
(51) Int. Cl.: B01F 23/231, B01F 23/233, B01F 27/191, B01F 27/2124, B01F 27/213, B01F 27/88, B01F 27/91, B01F 33/453, B01F 35/513

(54) **RECIPIENT-MELANGEUR ET PROCEDE D'ASSEMBLAGE D'UN RECIPIENT-MELANGEUR COMPRENANT UN ARBRE TELESCOPIQUE**
MISCHBEHÄLTER UND VERFAHREN ZUM AUFBAU EINES MISCHBEHÄLTERS MIT EINEM TELESKOPISCHEN WELLE
MIXING RECEPTACLE AND PROCESS FOR ASSEMBLING A MIXING RECEPTACLE COMPRISING A TELESCOPIC SHAFT

(30) Priorité: 03.08.2015 FR 1557500
(43) Date de publication de la demande: 13.06.2018
(73) Titulaire: Sartorius Stedim FMT, 13400 Aubagne (FR)
(72) Inventeur: CHAUSSIN, Sébastien, 13400 Aubagne (FR); GIBELIN, Jérémy, 83870 Signes (FR); ZEUCH, Stefan, 37079 Göttingen (DE); BATES, Michael, Stroud Gloucestershire GL5 2BL (GB)
(74) Mandataire: Novagraaf International SA
(86) Numéro de dépôt international: PCT/FR2016/052017
(87) Numéro de publication internationale: WO 2017/021653

(56) Documents cités:
- WO-A1-2013/151733
- WO-A1-2014/116165
- DE-A1- 102008 058 338
- DE-U1- 202007 005 868
- DE-U1- 202009 005 407

## Description

Elle vise plus particulièrement un procédé d'assemblage d'un récipient-mélangeur destiné à recevoir un fluide biopharmaceutique en vue de son mélange, ainsi qu'un tel récipient-mélangeur.

On entend par « fluide biopharmaceutique », un produit issu de la biotechnologie (milieux de cultures, cultures cellulaires, solutions tampon, liquides de nutrition artificielle, produits sanguins et dérivés de produits sanguins) ou un produit pharmaceutique ou plus généralement un produit destiné à être utilisé dans le domaine médical. L'invention s'applique également à des produits autres mais soumis à des exigences analogues en ce qui concerne leur conditionnement.

On connait des récipients-mélangeurs permettant le mélange de fluide biopharmaceutique. De tels récipients-mélangeurs comprennent un dispositif rigide extérieur de contention formant un logement destiné à recevoir un conteneur stérile à usage unique. Le conteneur comprend une paroi flexible délimitant un espace intérieur destiné à être empli du fluide biopharmaceutique. Le conteneur comprend également un organe de mélange fixé à un arbre descendant. L'arbre est fixé au conteneur un niveau d'un premier palier et d'un second palier. L'arbre du conteneur comprend au niveau du premier palier un disque comportant des aimants pouvant être placé en regard d'un disque similaire connecté à un moteur permettant ainsi l'entrainement magnétique en rotation de l'arbre sous l'effet du moteur. L'arbre peut ainsi tourner afin de permettre le mélange du fluide biopharmaceutique.

Un tel entrainement magnétique nécessite un alignement et un positionnement précis du disque magnétique de l'arbre et du disque magnétique du moteur pour assurer un entrainement optimal.

Toutefois, des problèmes de tolérances géométriques dues à la variabilité dimensionnelle inhérentes lors de la fabrication des éléments formant le récipient-mélangeur, peuvent conduire à un mauvais positionnement des disques magnétiques de l'arbre et du moteur en regard l'un de l'autre lors de l'installation du conteneur dans le dispositif rigide extérieur de contention. Par ailleurs, lors du mélange et du chauffage du fluide biopharmaceutique, par exemple à partir de températures de l'ordre de 30-40 degrés Celsius, une dilatation des pièces en matière plastique formant le récipient-mélangeur peuvent apparaitre. Ceci modifie l'agencement et la position du premier palier de l'arbre par rapport au moteur, conduisant à un mauvais fonctionnement du récipient-mélangeur. Il est alors nécessaire de pouvoir ajuster le positionnement du premier palier par rapport au moteur.

Il est ainsi connu d'utiliser un moteur dont la position est ajustable en hauteur. Toutefois, le réglage d'un tel moteur peut s'avérer difficile. Si le moteur est positionné trop bas, l'agencement du premier palier avec le moteur exerce une contrainte sur l'arbre, ce qui peut entrainer un fléchissement, voire la casse de l'arbre. En outre, un jeu de battement axial, de l'ordre de 2 millimètres, est nécessaire entre le premier palier et le disque magnétique du moteur pour permettre un fonctionnement satisfaisant du récipient-mélangeur. Un moteur fixé trop bas ne permet pas la présence de ce jeu de battement, ce qui génère de l'abrasion au niveau du premier palier lors du fonctionnement du récipient-mélangeur,

Au contraire, si le moteur est positionné trop haut, la paroi du conteneur se trouve sous contrainte en tension pour que le moteur et le premier palier puisse être positionnés l'un avec l'autre. Ces contraintes peuvent dégrader la paroi du conteneur, voire entrainer une déchirure résultant en une perte du fluide biopharmaceutique.

Ainsi, le réglage et le positionnement du moteur peut s'avérer long et compliqué à mettre en œuvre pour obtenir une installation satisfaisante du conteneur dans le dispositif rigide de contention.

**Le document** DE 20 2007 005868 U1 **propose d'utiliser, pour des bioréacteurs à usage unique, des agitateurs dont la longueur dépend du nombre d'éléments de tige superposés.**

**Dans le document** WO 2014/116165**, les contraintes susceptibles d'entrainer une déchirure en raison d'une agitation de type magnétique sont évitées par l'utilisation d'un ou deux supports portant chacun un palier associé à un arbre agitateur. Dans un exemple de réalisation avec deux supports et deux arbres interconnectés, une longueur totale de tige d'agitation peut être ajustée par serrage avant l'assemblage avec la paroi du conteneur enveloppant.**

On connait également des conteneurs comprenant des arbres de longueur variable, afin notamment de pouvoir replier le conteneur en raccourcissant la longueur de l'arbre et faciliter le stockage du conteneur.

A titre d'exemple, le document WO 2015/039034 décrit des structures de support de bioréacteur comprenant un arbre télescopique pouvant être utilisé avec des récipients de tailles et de formes diverses.

Le document US8951785 **(**DE 20 2008 058338 A1**)** décrit un agitateur pour un bioréacteur comportant une pluralité de bras articulés entre eux et pouvant pivoter autour d'un axe de rotation transversale. L'arbre peut ainsi avoir une hauteur ajustable en repliant les bras articulés.

Le document WO 2009/143925 **(ou** DE 20 2009 005407 U1**)** décrit un conteneur ayant deux éléments d'arbres adjacents comportant entre eux un corps creux dans lequel l'un des deux éléments de l'arbre peut coulisser. Un élément élastique est situé entre le corps creux et le corps de remplissage afin de permettre la transmission d'un mouvement de rotation entre les deux éléments. Une ouverture est ménagée dans la paroi du corps creux afin d'équilibrer la pression comprise dans le corps creux et dans le reste du conteneur. Une membrane hydrophobe perméable aux gaz est placée devant l'ouverture afin d'éviter que du fluide du conteneur puisse être introduit dans le corps creux.

Toutefois, un tel arbre est difficile à mettre en œuvre étant donné qu'il est nécessaire de prévoir une membrane hydrophobe sur l'ouverture du corps creux. En outre, dans le cas d'un arbre circulaire, l'élément élastique ne permet de transmettre que des couples de rotation peu élevés, empêchant un mélange efficace du fluide emplissant le conteneur.

L'invention vise à résoudre les inconvénients exposés ci-dessus et, plus particulièrement, vise à optimiser la mise en place du conteneur dans le dispositif rigide extérieur de contention afin de permettre un mélange satisfaisant du fluide biopharmaceutique.

A cet effet, selon un premier aspect, l'invention concerne un procédé d'assemblage d'un récipient-mélangeur destiné à recevoir un fluide biopharmaceutique en vue de son mélange, le procédé d'assemblage étant tel que défini dans la revendication 1.

Dans divers modes de réalisation selon la présente invention, on peut éventuellement avoir recours en outre à l'une et/ou à l'autre des dispositions suivantes, prises séparément ou en combinaison, selon lesquelles :
- le conteneur se trouve dans un état désassemblé vide de fluide biopharmaceutique lorsqu'on le place dans le dispositif rigide extérieur de contention ;
- la longueur de l'arbre est ajustable sur une course correspondant sensiblement à l'encombrement axial du moteur ;
- l'arbre est situé tout entier dans l'espace intérieur et dans lequel on ajuste la longueur de l'arbre selon l'axe principal en positionnant le premier palier en regard du moteur pour permettre au moteur d'entrainer l'arbre en rotation ; et
- le conteneur comprend également un second palier fixé à la paroi et on raccorde le second palier au dispositif rigide extérieur de contention après avoir placé le conteneur dans le dispositif rigide extérieur de contention.

Selon un deuxième aspect, l'invention concerne un récipient-mélangeur destiné à être assemblé par le procédé d'assemblage selon l'invention, le récipient-mélangeur étant tel que défini dans la revendication 8.

Dans divers modes de réalisation selon la présente invention, on peut éventuellement avoir recours en outre à l'une et/ou à l'autre des dispositions suivantes, prises séparément ou en combinaison, selon lesquelles :
- le moteur est fixe par rapport au dispositif rigide extérieur de contention ;
- le moteur est adapté pour permettre l'entrainement magnétique de l'arbre et comprend un disque rotatif menant situé à l'extérieur du conteneur, le disque rotatif menant coopérant fonctionnellement avec un disque rotatif mené fixé à l'arbre ;
- le premier palier comprend un flasque, le flasque comprenant une collerette annulaire extérieure, le moteur étant raccordé avec la collerette du flasque ; et
- on réalise une bioréaction, le récipient-mélangeur étant un bioréacteur.
Afin de former un récipient-mélangeur selon l'invention, on utilise un conteneur destiné à être assemblé à un moteur, selon le procédé d'assemblage.

Dans divers modes de réalisation selon la présente invention, on peut éventuellement avoir recours en outre à l'une et/ou à l'autre des dispositions suivantes, prises séparément ou en combinaison, selon lesquelles :
- l'arbre est situé tout entier dans l'espace intérieur ;
- l'arbre est traversant au niveau du premier palier ;
- l'arbre supporte et entraîne au moins un organe de mélange apte à mélanger le fluide biopharmaceutique situé dans l'espace intérieur ;
- l'arbre supporte et entraîne plusieurs organes de mélange situés en une pluralité de localisations axiales sur l'arbre ;
- le conteneur a une capacité comprise entre 50 litres et 200 litres ; et
- Le conteneur est à usage unique.
Le conteneur est pourvu d'un dispositif de mélange comprenant un arbre de longueur ajustable s'étendant entre un premier palier et un second palier, chacun des premier palier et second palier étant fixés à la paroi flexible du conteneur.

Dans divers modes de réalisation selon la présente invention, on peut éventuellement avoir recours en outre à l'une et/ou à l'autre des dispositions suivantes, prises séparément ou en combinaison, selon lesquelles :
- l'arbre comprend au moins une première partie et une deuxième partie mobiles en translation l'une par rapport à l'autre selon l'axe principal ;
- la fente traverse de part et d'autre la deuxième partie de l'arbre ;
- la fente a une longueur comprise entre 1 et 10 centimètres, voire égale à 5 centimètres ; et
- la première partie et la deuxième partie de l'arbre sont solidaires en rotation.

On décrit maintenant plusieurs modes de réalisation de l'invention à l'aide des dessins, dans lesquels :
- La figure 1 est une vue en perspective d'une réalisation possible d'un récipient-mélangeur selon l'invention montrant le dispositif rigide extérieur de contention ;
- La figure 2 est une autre vue en perspective du dispositif rigide extérieur de contention du récipient-mélangeur de la figure 1 ;
- La figure 3 est une vue de côté d'un conteneur destiné être à être placé dans le dispositif rigide extérieur de contention des figures 1 et 2 ;
- La figure 4 est une vue en coupe selon le plan IV-IV du conteneur de la figure 3, positionné par rapport à un moteur ;
- Les figures 5A et 5B sont des vues agrandies de l'arbre du dispositif de mélange au niveau du premier palier de la figure 4 selon deux agencements différents ; et
- La figure 6 est une vue schématique en coupe du conteneur placé dans le dispositif rigide extérieur de contention du récipient-mélangeur en état de fonctionnement, en particulier dans un état assemblé empli.

Un récipient-mélangeur 1 selon l'invention est destiné à recevoir un fluide biopharmaceutique C, en vue de son mélange ou, le cas échéant en vue d'une réaction chimique et/ou biologique (ou bioréaction), le récipient-mélangeur 1 étant alors un bioréacteur.

Le fluide biopharmaceutique C comprend une ou au moins une phase liquide. Le cas échéant, le fluide biopharmaceutique C est réalisé à partir de plusieurs composants dont au moins un est en phase liquide et dont un ou plusieurs peut être en phase solide, tel que de la poudre.

Le récipient-mélangeur 1 présente un axe principal XX, vertical. Le récipient-mélangeur 1 comporte d'une part un conteneur 2 et d'autre part un dispositif rigide extérieur de contention 18.

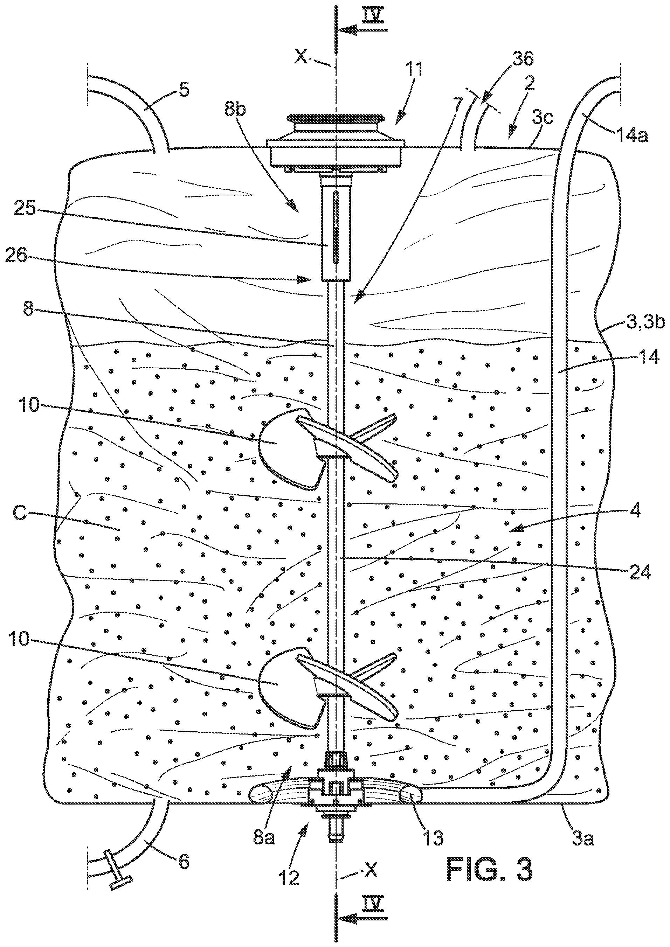

Comme représenté sur la figure 3, le conteneur 2 est formé par une paroi 3, avantageusement en matière plastique, flexible et étanche au fluide biopharmaceutique C. La paroi 3 du conteneur 2 peut comprendre une partie inférieure 3a, une partie latérale 3b et une partie supérieure 3c, par exemple formée par un ou plusieurs tronçons solidarisés, soudés, les uns avec les autres. Le conteneur 2 délimite ainsi un espace intérieur 4, avantageusement stérile, apte à recevoir une certaine quantité du fluide biopharmaceutique C. La paroi 3 peut être totalement ou partiellement transparente ou translucide afin de pouvoir visualiser depuis l'extérieur le fluide biopharmaceutique C dans l'espace intérieur 4.

Selon une réalisation, le conteneur 2 est à usage unique.

Le conteneur 2 peut avoir une capacité allant jusqu'à 5 000 litres, en fonction des besoins et des applications. Le conteneur 2 a cependant de préférence une capacité comprise entre 10 et 500 litres, voire entre 50 et 200 litres.

Les mots « vertical », « horizontal », « supérieur », « inférieur » se réfèrent à la situation dans laquelle le récipient-mélangeur 1, et en particulier le conteneur 2, est dans une position apte à son fonctionnement. Il est entendu toutefois que le récipient-mélangeur 1 et le conteneur 2 peuvent occuper d'autres positions ou avoir d'autres états, par exemple parce qu'ils ne sont pas en fonctionnement. Le mot « vertical » ne doit pas être compris dans un sens étroit, mais dans le sens signifiant du plus haut au plus bas et inversement.

D'autre part, les mots « intérieur » et « extérieur » se réfèrent, respectivement à ce qui se trouve dans l'espace intérieur 4 et hors du conteneur 2.

Enfin, les mots « axial » d'une part, « radial » et « transversal » d'autre part, se réfèrent à ce qui s'étend dans ou parallèlement ou sensiblement parallèlement à l'axe principal XX d'une part, et perpendiculairement ou orthogonalement ou sensiblement perpendiculairement ou orthogonalement à l'axe principal XX d'autre part.

Le récipient-mélangeur 1 peut comporter un ou plusieurs ports 5 traversant d'introduction dans le conteneur 2 du fluide biopharmaceutique C, ou de composants du fluide biopharmaceutique C, coopérant avec un ou plusieurs orifices d'introduction ménagés dans le conteneur 2.

Le récipient-mélangeur 1 peut comporter également au moins un port 6 traversant de vidange du fluide biopharmaceutique C du conteneur 2, coopérant avec au moins un orifice de vidange ménagé dans le conteneur 2. Le port de vidange 6 est apte à être obturé à chaque fois que nécessaire et au contraire ouvert pour la vidange.

On entend par « port », un moyen de connexion ou de liaison physique. Un tel port est traversant lorsqu'il s'agit d'assurer une fonction de mise en communication entre l'espace intérieur 4 et l'extérieur du conteneur 2, par exemple pour l'introduction ou la vidange de ce qui doit être placé ou est placé dans le conteneur 2. Un tel port peut également être non traversant lorsqu'il s'agit d'assurer une fonction de maintien d'un organe du récipient-mélangeur.

Des conduits, poches, réservoirs, le cas échéant souples, peuvent être associé au port d'introduction 5, en communication fluidique et avec une connexion étanche et le cas échéant amovible. De même, des conduits, poches, réservoirs, le cas échéant souples peuvent être associé au port de vidange 6, en communication fluidique et avec une connexion étanche et le cas échéant amovible.

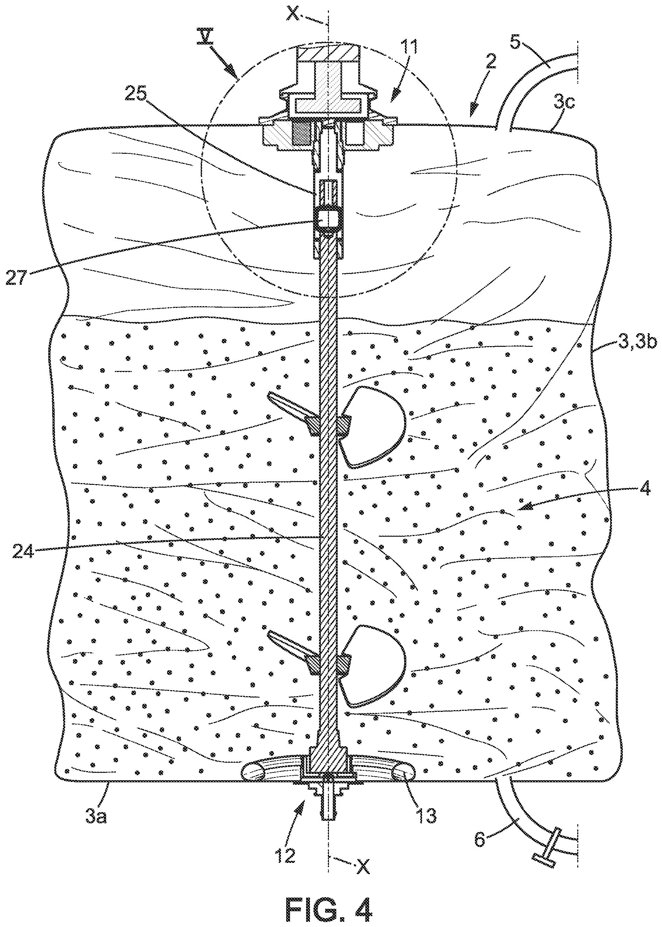

Dans la réalisation représentée sur les figures 3 et 4, le port d'introduction 5 est situé dans la partie supérieure 3c de la paroi 3, tandis que le port de vidange 6 est situé dans la partie inférieure 3a du conteneur 2, en particulier en position la plus basse du récipient-mélangeur 1. Cependant, cette réalisation n'est pas limitative et un ou plusieurs ports d'introduction 5 peuvent être situés dans la partie inférieure 3a ou dans la partie latérale 3b du conteneur 2.

Le récipient-mélangeur 1 peut également comporter un dispositif d'aération 13 apte à délivrer au fluide biopharmaceutique C une certaine quantité de gaz d'aération. Ce dispositif 13 permet ainsi l'aération de ce qui se trouve dans l'espace intérieur 4 du conteneur 2, qu'il s'agisse du fluide biopharmaceutique C, ou d'une partie de ses composants.

Le dispositif d'aération 13 peut comprendre un dispositif d'amenée de gaz d'aération 14 ayant au moins un élément tubulaire 14a s'étendant avec communication fluidique depuis l'extérieur du conteneur 2. Au dispositif d'aération 13 qui vient d'être décrit peut être associé fonctionnellement au moins un port d'évacuation de gaz d'aération 36 ménagé dans la partie supérieure 3c de la paroi 3 du conteneur 2. Un tel port d'évacuation de gaz d'aération 36 permet d'évacuer du conteneur 2, vers l'extérieur, le gaz qui n'a pas été mélangé avec le fluide biopharmaceutique C du conteneur 2.

Le récipient-mélangeur 1 peut, dans certaines réalisations, comporter également d'autres ports connus en soi, par exemple de montage d'un moyen fonctionnel, apte à assurer le maintien d'un organe tel que typiquement la collecte ou la mesure de données, ou la prise d'échantillon aux fins d'analyse.

Le récipient-mélangeur 1 comporte également un dispositif de mélange 7 du fluide biopharmaceutique C du conteneur 2. Ce dispositif de mélange 7 permet le mélange de ce qui se trouve dans l'espace intérieur 4 du conteneur 2, qu'il s'agisse du fluide biopharmaceutique C, ou d'une partie de ses composants.

Le dispositif de mélange 7 comprend au moins un arbre 8 descendant, apte à être entraîné, notamment magnétiquement, en rotation par un moteur 9 et à entraîner en rotation au moins un organe de mélange 10. Le ou les organes de mélange 10 sont substantiellement espacés de la partie inférieure 3a et de la partie latérale 3b de la paroi 3 du conteneur 2. Comme représenté sur les figures 3, 4 et 6, l'organe de mélange 10 peut se présenter sous la forme d'une hélice ayant un moyeu supportant plusieurs pales.

L'arbre 8 est ajustable en longueur. Selon la réalisation représentée sur les figures, l'arbre 8 est ainsi formé de deux parties 24, 25. Une première partie 24 s'étend entre l'extrémité inférieure 8a jusqu'à une zone de liaison intermédiaire 26, tandis que la deuxième partie 25 s'étend depuis la zone de liaison 26 jusqu'à l'extrémité supérieure 8b.

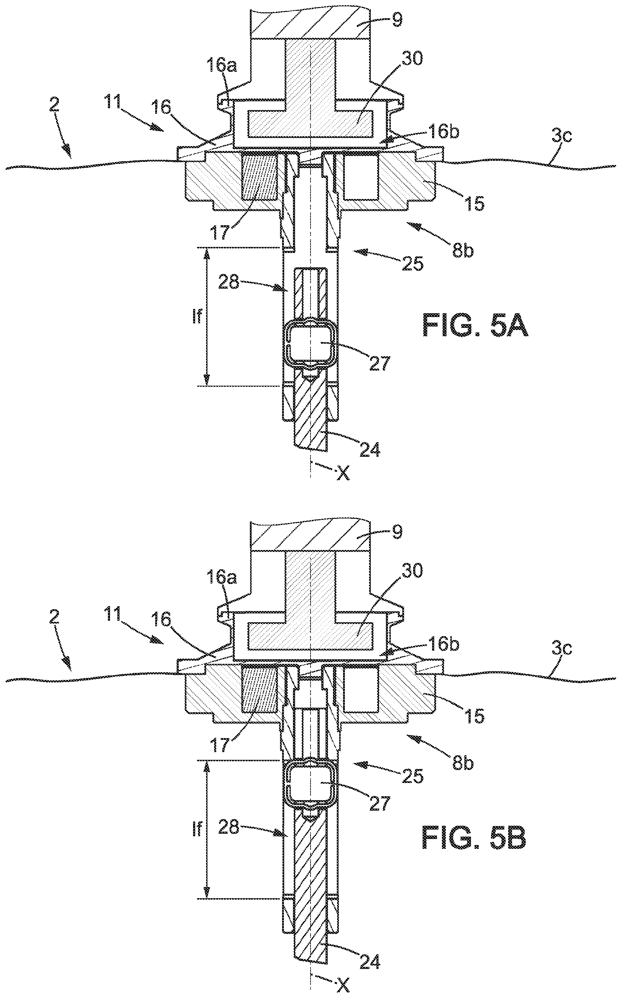

Comme représenté plus en détails sur les figures 5A et 5B, la première partie 24 de l'arbre 8 au niveau de la zone de liaison 26 comprend un élément 27, telle qu'une clavette, logé dans une fente 28 de la deuxième partie 25 de l'arbre 8. La fente 28 s'étend notamment de façon rectiligne selon l'axe principal XX. La fente 28 traverse notamment de part et d'autre la deuxième partie 25 de l'arbre 8. L'élément 27 a alors notamment une forme aplatie, notamment carrée, afin de passer dans la fente 28 traversant la deuxième partie 25 de l'arbre 8.

La première partie 24, notamment l'élément 27, est adapté pour coulisser dans la deuxième partie 25 de l'arbre 8 selon l'axe principal XX. Comme représentée sur les figures 5A et 5B, la fente 28 a une longueur l_{f}, par exemple comprise entre 1 et 10 centimètres, de préférence égale à environ 5 centimètres. En particulier, la longueur l_{f} peut être adaptée en fonction de la taille du conteneur 2. Ainsi, l'arbre 8 est ajustable en longueur entre une position complètement allongée et une position complètement rétractée. Dans la position complètement rétractée, comme représenté sur la figure 5B, l'arbre 8 a une hauteur minimale, la première partie 24, en particulier l'élément 27, venant en butée avec la deuxième partie 25 de l'arbre 8.

L'élément 27 est adapté pour coulisser de façon continue dans la fente 28. L'arbre 8 peut donc avoir une longueur ajustable de façon continue et non discrète, par exemple en cas de dilatation de certains éléments du récipient-mélangeur 1 en cours de mélange.

En outre, du fait que l'élément 27 fait saillie dans la fente 28, la première partie 24 et la deuxième partie 25 de l'arbre 8 sont solidaires en rotation, notamment lorsqu'ils sont soumis à des couples de torsion élevée.

En outre, du fait que l'arbre 8 a une longueur ajustable, le moteur 9 peut être fixe par rapport au dispositif extérieur de contention 18, et il n'est pas nécessaire que celui-ci ait un positionnement ajustable, notamment en hauteur afin de placer l'arbre 8 en regard du moteur 9 pour permettre la rotation de l'arbre 8 comme cela sera décrit ci-après.

Le conteneur 2 comprend également au moins un premier palier 11, adjacent à la partie supérieure 3c de la paroi 3, avec lequel coopère la partie supérieure 8b de l'arbre 8.

Le premier palier 11 comporte un flasque rigide 16. On entend ici par « flasque », une pièce rigide en forme générale de paroi pleine, au moins sensiblement plate, placée à plat, et destinée au maintien. Ce flasque 16 est fixé de façon rigide et étanche à la partie supérieure 3c de la paroi 3 du conteneur 2.

Plus précisément, le flasque 16 est formé d'une matière sensiblement rigide, de préférence une matière plastique rigide, en forme de paroi ou de plaquette raccordée au conteneur 2, au centre de la partie supérieure 3c. Ce flasque 16 peut être raccordé à la paroi 3 du conteneur 2 de toute manière appropriée de façon à former un joint rigide et hermétique entre les matières respectives, rigide et flexible du flasque 16 et de la paroi 3.

Selon un premier mode de réalisation, l'arbre 8 du dispositif de mélange 7 est situé tout entier dans l'espace intérieur 4. Ainsi, l'arbre 8 s'étend de façon rectiligne entre une extrémité inférieure 8a et une extrémité supérieure 8b. Lorsque le récipient-mélangeur 1 est dans une position apte à son fonctionnement, l'arbre 8 s'étend de façon verticale suivant l'axe principal XX, l'extrémité inférieure 8a étant située vers la partie inférieure 3a du conteneur 2 tandis que l'extrémité supérieure 8b est située vers la partie supérieure 3c du conteneur 2 en étant reliée au premier palier 11. Le premier palier 11 est alors adapté pour être positionné par rapport au moteur 9 situé à l'extérieur du conteneur 2.

Selon le premier mode de réalisation représenté par exemple sur les figures 5A et 5B, le moteur 9 d'entraînement permet l'entrainement magnétique en rotation de l'arbre 8. A cet effet, le moteur 9 comprend un disque rotatif menant 30 situé à l'extérieur du conteneur 2. L'arbre 8 comprend alors un disque rotatif mené 15 destiné à coopérer fonctionnellement, notamment magnétiquement, avec le disque rotatif menant 30 du moteur 9. Plus particulièrement, le disque rotatif mené 15 comprend une pluralité d'aimants 17, qui sont intégrés par tout moyen de fixation ou de construction, afin de permettre la rotation de l'arbre 8 lors de la rotation du disque rotatif menant du moteur 9.

Le disque rotatif mené 15 est solidaire, notamment en rotation, de l'arbre 8, en particulier de la deuxième partie 25 de l'arbre 8. Par exemple, le disque rotatif mené 15 est fixé à l'extrémité supérieure 8b de l'arbre 8 par vissage d'une extrémité filetée de l'arbre 8 dans une ouverture filetée à l'intérieur du disque rotatif mené 15. D'autres moyens, tels qu'éléments adhésifs, attaches, fixations rapides, verrous, soudage, et similaires, ainsi que la formation du disque rotatif mené 15 directement par moulage avec l'arbre 8 lors de sa fabrication, peuvent être utilisés pour fixer le disque rotatif mené 15 à l'arbre 8, sans limitation.

En outre, le disque rotatif mené 15 est raccordé au premier palier 11, notamment au flasque 16, de manière à permettre au moteur 9 d'agir sur les aimants 17 du disque rotatif mené 15. Ainsi, le flasque 16 est relié à l'arbre 8 dans l'espace intérieur 4 du conteneur 2 par l'intermédiaire du disque rotatif mené 15. En particulier, l'arbre 8 et le disque rotatif mené 15 sont montés mobiles en rotation autour de l'axe principal XX par rapport au premier palier 11, de sorte que le disque rotatif mené 15 peut tourner relativement librement par rapport au flasque 16. A cet effet, on peut prévoir d'inclure des éléments de roulement à billes ou à rouleaux entre le disque rotatif mené 15 et le premier palier 11.

En état de fonctionnement, le premier palier 11 est positionné, en particulier assemblé, par rapport au moteur 9. Le disque rotatif menant 30 du moteur 9 et le disque rotatif mené 15 sont alors disposé en regard l'un de l'autre, de chaque côté du premier palier 11. Il peut être prévu un jeu de battement entre le disque rotatif menant 30 et le premier palier 11, par exemple de l'ordre de 2 millimètres, afin de faciliter la rotation du premier palier 11 par rapport au moteur 9.

Le premier palier 11, notamment le flasque 16, comprend par exemple une collerette annulaire extérieure 16a comportant un bourrelet radial terminal s'étendant latéralement vers l'extérieur et limitant vers l'intérieur une cavité 16b du flasque 16. Le moteur 9 peut être positionné par rapport au flasque 16 et notamment avec la collerette 16a de manière fixe en translation. En particulier, le disque rotatif menant 30 du moteur 9 est destiné à être disposé dans la cavité 16b du flasque 16 comme cela est représenté sur les figures 5A et 5B.

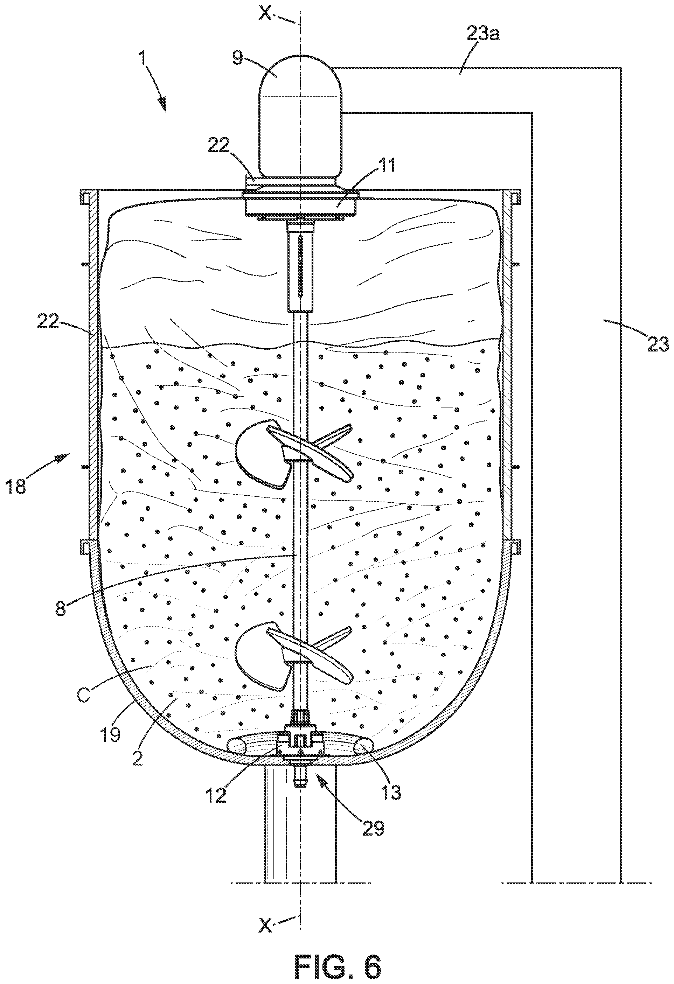

Comme représenté sur la figure 6, une pince 22 faisant fonction de bride permet de connecter le flasque 16 avec le moteur 9. Une telle pince comprend classiquement une menotte de serrage et est connue sous le nom de pince « tri-clamp ». La pince 22 est apte et destinée à venir se serrer sur le moteur 9 et la collerette 16a du flasque 16 en les maintenant solidaire en translation pour éviter leur désassemblage intempestif. Toutefois, le moteur 9 reste mobile en rotation par rapport au premier palier 11 autour de l'axe principal XX. Ainsi, le disque rotatif menant 30 du moteur 9 peut entrainer en rotation le disque rotatif mené 15.

Selon un deuxième mode de réalisation non représenté sur les figures, l'arbre 8 peut être partiellement situé à l'extérieur du conteneur 2. Selon ce mode de réalisation, l'arbre 8 traverse le conteneur 2, en particulier au niveau du premier palier 11 de façon étanche. Le disque rotatif mené 15 de l'arbre est alors situé à l'extérieur du conteneur 2 et est destiné à coopérer fonctionnellement, notamment magnétiquement, avec le disque rotatif menant 30 du moteur 9.

Selon ce mode de réalisation, la zone de liaison 26 de l'arbre 8 peut être située à l'extérieur du conteneur 2. La longueur de l'arbre 8 peut ainsi être facilement ajustée depuis l'extérieur du conteneur 2 par l'utilisateur du récipient-mélangeur 1, ce qui permet d'obtenir un conteneur 2 simple d'utilisation et économique à réaliser. En outre, la zone de liaison 26 est alors plus facile d'accès, ce qui permet de faciliter sa stérilisation avant utilisation du conteneur 2.

Le récipient-mélangeur 1 peut comporter également, en raison de la nature flexible du conteneur 2, un dispositif rigide extérieur de contention 18 pour une contention du conteneur 2 empli de son fluide biopharmaceutique C pendant le remplissage, le mélange et la vidange.

Le dispositif rigide extérieur de contention 18 comprend une paroi de fond 19 et une paroi périphérique 20 délimitant un logement dans lequel est placé de façon amovible le conteneur 2. La paroi de fond 19 a par exemple une forme de calotte arrondie, par exemple hémisphérique ou pseudo hémisphérique, Toutefois, le dispositif rigide extérieur de contention 18 peut avoir toute autre forme, telle que cylindrique, parallèpipédique ou autres.

La partie inférieure 3a de la paroi 3 du conteneur 2 repose sur la paroi de fond 19, tandis que la partie latérale 3b de la paroi 3 du conteneur 2 vient s'appliquer, lorsque le conteneur 2 est empli de fluide biopharmaceutique C, contre la paroi périphérique 20. Le dispositif rigide extérieur de contention 18 est généralement de géométrie, forme et et/ou dimension identiques au conteneur 2, afin de réduire les sollicitations ou les contraintes mécaniques sur la paroi 3 du conteneur 2.

Le dispositif rigide extérieur de contention 18 peut comporter une ouverture d'accès 21 afin de permettre la mise en place et l'enlèvement du conteneur 2. Le cas échéant, le dispositif rigide extérieur de contention 18 comporte un moyen de fermeture, tels que des portes, afin de permettre alternativement l'ouverture ou la fermeture de l'ouverture d'accès 21.

Selon une réalisation, le dispositif rigide extérieur de contention 18 comporte d'autres ouvertures pour introduire le fluide biopharmaceutique C ou les composants du fluide biopharmaceutique C et vidanger le fluide biopharmaceutique C, ou pour accéder aux différents éléments du conteneur 2 qui doivent être accessibles pour l'utilisation.

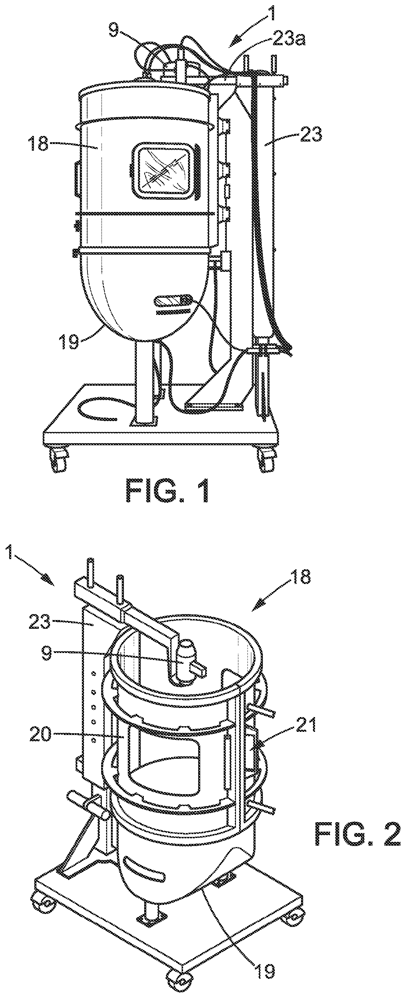

Le moteur 9 est avantageusement placé au-dessus du dispositif rigide extérieur de contention 18, de façon fixe. Comme représenté plus particulièrement sur les figures 1 et 2, le moteur 9 peut être notamment fixé à un bras de maintien 23 comportant une partie transversale 23a. Du fait de cette partie transversale 23a du bras de maintien 23, le moteur 9 est plus particulièrement centré au-dessus du dispositif rigide extérieur de contention 18 selon l'axe principal XX.

Selon une réalisation, le dispositif rigide extérieur de contention 18 comporte également un dispositif de chauffage et/ou de refroidissement destiné au chauffage et/ou au refroidissement du fluide biopharmaceutique C du conteneur 2. Dans ce cas, le dispositif rigide extérieur de contention 18 et/ou le conteneur 2 sont réalisés en un matériau présentant une certaine conductivité thermique, de manière que la mise en œuvre du dispositif de chauffage et/ou de refroidissement permette le chauffage et/ou le refroidissement du fluide biopharmaceutique C. Dans ce cas, et le cas échéant, il est également prévu un dispositif de contrôle de la température du contenu dans le conteneur 2 et un dispositif de commande du dispositif de chauffage et/ou de refroidissement. Un tel dispositif de contrôle de la température peut être porté par un ou des ports prévus à cet effet.

Le conteneur 2 peut comprendre un unique premier palier 11, pour être positionné par rapport au moteur 9. Toutefois, en variante, le conteneur 2 peut en outre comprendre un second palier 12, adjacent à la partie inférieure 3a de la paroi 3, avec lequel coopère la partie inférieure 8a de l'arbre 8. De la même façon que le premier palier 11, le second palier 12 est raccordé à la paroi 3 du conteneur 2 pour former un joint rigide et étanche avec la partie inférieure 3a de la paroi 3. A cet effet, le second palier 12 comprend un flasque (ce terme devant être compris comme précédemment) fixé de façon rigide et étanche à la partie inférieure 3a de la paroi 3 du conteneur 2.

Le conteneur 2 est alors raccordé au niveau du second palier 12 au dispositif extérieur de contention 18. Selon le premier mode de réalisation décrit ci-dessus dans lequel l'arbre 8 du dispositif de mélange 7 est situé tout entier dans l'espace intérieur 4, il est ainsi possible d'ajuster la taille du conteneur 2 en ajustant la longueur de l'arbre 8 qui s'étend entre le premier palier 11 et le second palier 12.

Le conteneur 2 peut se trouver dans trois états distincts en relation avec le dispositif rigide extérieur de contention 18 :
- un état désassemblé vide, dans lequel le conteneur 2 est désassemblé du dispositif rigide extérieur de contention 18 et n'est pas positionné par rapport au moteur 9. Dans cet état, le conteneur 2, qui est flexible dans son ensemble, alors qu'il est vide du fluide biopharmaceutique C, peut être disposé de façon aplatie sur lui-même. Cet état est tout particulièrement utile pour le stockage ou le transport ;
- un état assemblé vide, dans lequel le conteneur 2 est assemblé au dispositif rigide extérieur de contention 18, le conteneur 2 étant vide du fluide biopharmaceutique C. Dans cet état, le conteneur 2 est disposé dans le logement du dispositif de contention 18 en reposant sur la paroi de fond 19. On entend notamment par « assemblage », le fait que le conteneur 2 interagit fonctionnellement avec le moteur 9. Toutefois, un tel assemblage n'est pas limité à un contact direct entre le conteneur 2 et le moteur 9, ces éléments pouvant être assemblés selon l'invention tout en étant espacés l'un de l'autre, par exemple dans le cadre d'un entrainement magnétique ; et
- et, enfin, un état assemblé partiellement ou complètement empli, dans lequel le conteneur 2 est assemblé au dispositif rigide extérieur de contention 18 et positionné par rapport au moteur 9, le conteneur 2 étant empli de fluide biopharmaceutique C. Dans cet état, le récipient-mélangeur 1 est apte à fonctionner afin de permettre le mélange du fluide biopharmaceutique C.

On décrit ci-après le procédé d'assemblage d'un récipient-mélangeur 1 selon le premier mode de réalisation, notamment pour passer entre les différents états du conteneur 2 décrits ci-dessus.

On part d'un récipient-mélangeur 1 dont le conteneur 2 est dans un état désassemblé du dispositif rigide extérieur de contention 18, ainsi que vide de fluide biopharmaceutique C et disposé de façon plus ou moins aplatie sur lui-même.

On dispose le conteneur 2 dans le logement au dispositif rigide extérieur de contention, en reposant sur sa paroi de fond 19.

On raccorde le second palier 12 du conteneur 2 avec le dispositif rigide extérieur de contention 18, par exemple avec une ouverture 29 située au centre de la paroi de fond 19.

On positionne ensuite le premier palier 11 du conteneur 2 par rapport au moteur 9. On amène donc la poche 3 du conteneur 2 au niveau du moteur 9.

L'arbre 8 se trouve alors dans une position au moins partiellement rétractée. Du fait que l'arbre 8 soit ajustable en longueur, il est possible tout d'abord de placer l'extrémité supérieure 8b, notamment le premier palier 11, écarté du moteur 9. Le premier palier 11 peut notamment être placé au voisinage immédiat du moteur 9, par exemple à une distance du moteur 9 inférieure à la longueur l_{f} de la fente 28. Le disque rotatif mené 15 est alors situé face au moteur 9. On augmente ensuite la longueur de l'arbre 8 afin que le premier palier 11 soit positionné en regard du moteur 9, notamment raccordé sans frottement et avec un jeu de battement entre le disque rotatif menant 30 du moteur 9 et le premier palier 11, de sorte que le moteur 9 peut tourner facilement.

Afin d'ajuster la longueur de l'arbre 8, le coulissement des deux parties 24, 25 de l'arbre 8 entre elles peut être réalisé manuellement ou avec tout autre outil permettant un tel coulissement. Ainsi, il n'est pas nécessaire lors de connexion du premier palier 11 avec le moteur 9, de faire varier la position du moteur 9. Le conteneur 2 est ainsi assemblé plus facilement et de façon optimale avec le dispositif rigide extérieur de contention 18.

Le moteur 9 est raccordé avec la collerette 16a du flasque 16 de façon solidaire en translation grâce à la pince 22 (menotte tri-clamp) faisant fonction de bride. Ainsi agencé, le disque rotatif menant du moteur 9 est apte à entrainer en rotation le disque rotatif mené 15, et donc l'arbre 8 du dispositif de mélange 7. La figure 6 représente notamment le conteneur 2 ainsi agencé avec le dispositif rigide extérieur de contention 18 et le moteur 9.

Alternativement, il est possible de connecter le premier palier 11 du conteneur 2 avec le moteur 9 avant de raccorder le second palier 12 avec le dispositif rigide extérieur de contention 18.

On introduit dans le conteneur 2 le fluide biopharmaceutique C, notamment grâce au port d'introduction 5.

Enfin, on met en œuvre le dispositif de mélange 7 pour agiter le fluide biopharmaceutique C du conteneur 2 se trouvant dans l'espace intérieur 4. Le cas échéant, la longueur de l'arbre s'adapte pour garantir le positionnement relatif optimal du moteur 9 et du premier palier 11.

Par ailleurs, dans le cadre d'un procédé de bioréaction on met en œuvre le dispositif d'aération 13 pour délivrer au contenu du conteneur 2 se trouvant dans l'espace intérieur 4, une certaine quantité de gaz d'aération. L'agitation et l'aération sont réalisées au moins partiellement simultanément, le cas échéant, totalement simultanément.

Suite au mélange du fluide biopharmaceutique C et à sa vidange, notamment par le port de vidange 6, on désassemble le conteneur 2 du dispositif rigide extérieur de contention 18. On jette alors le conteneur 2, celui-ci étant à usage unique.

Le procédé décrit ci-dessus peut être réalisé partiellement, les étapes décrites ci-dessus pouvant être réalisée indépendamment l'une de l'autre. En particulier, le conteneur 2 peut être disposé dans le dispositif rigide extérieur de contention 18 alors qu'il est déjà empli de fluide biopharmaceutique C.

Bien évidemment, l'invention n'est pas limitée aux modes de réalisation décrits précédemment et fournis uniquement à titre d'exemple. Elle englobe diverses modifications, formes alternatives et autres variantes que pourra envisager l'homme du métier dans le cadre de la présente invention telle que revendiquée et notamment toutes combinaisons des différents modes de fonctionnement décrits précédemment, pouvant être pris séparément ou en association.

## Revendications

1. Procédé d'assemblage d'un récipient-mélangeur (1) destiné à recevoir un fluide biopharmaceutique (C) en vue de son mélange, dans lequel :
- on dispose d'un conteneur (2) comportant une paroi flexible (3) délimitant un espace intérieur (4) apte à être rempli de fluide biopharmaceutique (C), le conteneur (2) comprenant :
o un dispositif de mélange (7) comportant un arbre (8) présentant une longueur ajustable selon un axe principal (XX), l'arbre (8) s'étendant de façon rectiligne entre une extrémité inférieure (8a) et une extrémité supérieure (8b),
o un premier palier (11) fixé à la paroi (3), l'arbre (8) s'étendant au moins dans l'espace intérieur (4) depuis le premier palier (11),
- on dispose d'un dispositif rigide extérieur de contention (18) du conteneur (2),
- on dispose d'un moteur d'entraînement (9) situé à l'extérieur du conteneur (2), le moteur (9) étant adapté pour entrainer en rotation l'arbre (8) du dispositif de mélange (7),
- on place le conteneur (2) dans le dispositif rigide extérieur de contention (18), le dispositif rigide extérieur de contention (18) comprenant une paroi de fond (19) et une paroi périphérique (20) délimitant un logement adapté pour recevoir le conteneur (2), la paroi flexible (3) du conteneur (2) étant disposée sur la paroi de fond (19) du dispositif rigide extérieur de contention (18), et
- sachant que la position du moteur est fixe par rapport au dispositif rigide extérieur de contention (18) et que le conteneur (2) est déjà placé dans le dispositif rigide extérieur de contention (18), on ajuste la longueur de l'arbre (8) selon l'axe principal (XX) en disposant l'arbre (8) en regard du moteur (9), du côté de l'extrémité supérieure (8b), pour permettre au moteur (9) d'entrainer l'arbre (8) en rotation, l'arbre (8) s'étendant de façon verticale suivant l'axe principal (XX) avec l'extrémité inférieure (8a) située vers une partie inférieure (3a) du conteneur (2) tandis que l'extrémité supérieure (8b) est située vers une partie supérieure (3c) du conteneur (2) en étant reliée au premier palier (11),
et dans lequel l'arbre (8) présente une première partie (24) et une deuxième partie (25) qui sont mobiles en translation l'une par rapport à l'autre selon l'axe principal (XX), la première partie (24) comprenant un élément (27) adapté pour coulisser dans une fente rectiligne (28) de la deuxième partie (25) de l'arbre en ajustant la longueur de l'arbre (8), et dans lequel le premier palier (11) est placé au voisinage immédiat du moteur (9), à une distance du moteur (9) inférieure à la longueur (If) de la fente rectiligne (28).

2. Procédé selon la revendication 1, dans lequel le moteur (9) est adapté pour entrainer en rotation l'arbre (8), par entraînement magnétique en rotation dudit arbre (8), le premier palier (11) comportant un flasque (16) rigide fixé de façon rigide et étanche à une partie supérieure (3c) de la paroi (3) du conteneur (2),
et dans lequel on amène la partie supérieure (3c) de la paroi flexible (3) au niveau du moteur (9) afin qu'un disque rotatif menant (30) appartenant au moteur (9) et situé à l'extérieur du conteneur (2) soit disposé dans une cavité (16b) du flasque (16), le disque rotatif menant (30) coopérant fonctionnellement avec un disque rotatif mené (15) fixé à l'arbre (8) à l'extrémité supérieure (8b), le disque rotatif mené (15) comprenant une pluralité d'aimants (17) et étant apte à tourner par rapport au flasque (16).

3. Procédé selon la revendication 1 ou 2, dans lequel le conteneur (2) se trouve dans un état désassemblé vide de fluide biopharmaceutique (C) lorsqu'on le place dans le dispositif rigide extérieur de contention (18).

4. Procédé selon la revendication 1, 2 ou 3, dans lequel la longueur de l'arbre (8) est ajustable sur une course correspondant sensiblement à l'encombrement axial du moteur (9).

5. Procédé selon l'une quelconque des revendications 1 à 4, dans lequel l'arbre (8) est situé tout entier dans l'espace intérieur (4) et on ajuste la longueur de l'arbre (8) selon l'axe principal (XX) en positionnant le premier palier (11) en regard du moteur (9) pour permettre au moteur (9) d'entrainer l'arbre (8) en rotation.

6. Procédé selon l'une quelconque des revendications précédentes, dans lequel le conteneur (2) comprend également un second palier (12) fixé à la paroi (3) et on raccorde le second palier (12) au dispositif rigide extérieur de contention (18) après avoir placé, via une ouverture d'accès (21), le conteneur (2) dans le dispositif rigide extérieur de contention (18), l'ouverture d'accès (21) étant prévue dans la paroi périphérique (20) et permettant la mise en place et l'enlèvement du conteneur (2).

7. Procédé selon l'une quelconque des revendications précédentes, dans lequel le conteneur (2) pourvu d'un port de vidange (6) est placé de façon amovible dans le dispositif rigide extérieur de contention (18), ledit conteneur (2) étant à usage unique.

8. Récipient-mélangeur (1) destiné à être assemblé par le procédé d'assemblage selon l'une quelconque des revendications précédentes, comprenant :
- ledit conteneur (2) dont l'espace intérieur (4) délimité par la paroi flexible (3) est apte à être rempli de fluide biopharmaceutique (C), le conteneur (2) comprenant :
o le dispositif de mélange (7) comportant l'arbre (8) qui s'étend de façon rectiligne et dont la longueur est ajustable selon un axe principal (XX),
o le premier palier (11) depuis lequel l'arbre (8) s'étend dans l'espace intérieur (4),
- le moteur d'entraînement (9) situé à l'extérieur du conteneur (2), le moteur (9) étant adapté pour entrainer en rotation l'arbre (8) du dispositif de mélange (7), , et
- le dispositif rigide extérieur de contention (18) dont la paroi de fond (19) et la paroi périphérique (20) délimitent le logement adapté pour recevoir le conteneur (2), **caractérisé en ce que** le moteur (9) est fixe par rapport au dispositif rigide extérieur de contention (18), la longueur de l'arbre (8) étant ajustable selon l'axe principal (XX) afin que l'arbre (8) s'étende de façon verticale suivant l'axe principal (XX) en présentant ladite extrémité inférieure (8a) située vers une partie inférieure (3a) du conteneur (2) et ladite extrémité supérieure (8b) située vers la partie supérieure (3c) du conteneur (2) en étant reliée au premier palier (11),
dans lequel l'arbre (8) de longueur ajustable s'étend entre le premier palier (11) et un second palier (12), chacun des premier palier (11) et second palier (12) étant fixés à la paroi (3) flexible dudit conteneur (2),
et dans lequel l'arbre (8) comprend une première partie (24) et une deuxième partie (25) mobiles en translation l'une par rapport à l'autre selon l'axe principal (XX), la première partie comprenant un élément (27) adapté pour coulisser dans une fente rectiligne (28) de la deuxième partie (25) de l'arbre (8).

9. Récipient-mélangeur (1) selon la revendication 8, dans lequel le moteur (9) est adapté pour permettre l'entrainement magnétique de l'arbre (8) et comprend un disque rotatif menant (30) situé à l'extérieur du conteneur (2), le disque rotatif menant (30) coopérant fonctionnellement avec un disque rotatif mené (15) fixé à l'arbre (8).

10. Récipient-mélangeur (1) selon la revendication 9, dans lequel le premier palier (11) comporte un flasque (16) rigide fixé de façon rigide et étanche à une partie supérieure (3c) de la paroi (3) du conteneur (2), le flasque (16) comprenant une collerette annulaire extérieure (16a) avec laquelle le moteur (9) est raccordé au conteneur (2), et dans lequel le flasque (16) est relié à l'arbre (8) dans l'espace intérieur (4) du conteneur (2) par l'intermédiaire du disque rotatif mené (15), le disque rotatif mené (15) pouvant tourner relativement librement par rapport au flasque (16).

11. Récipient-mélangeur (1) selon l'une quelconque des revendications 8 à 10, dans lequel l'arbre (8) supporte et entraîne au moins un organe de mélange (10) apte à mélanger le fluide biopharmaceutique (C) situé dans l'espace intérieur (4).

## Patentansprüche

1. Verfahren zum Zusammenbauen eines Mischbehälters (1), der bestimmt ist, ein biopharmazeutisches Fluid (C) zum Mischen aufzunehmen, wobei:
- ein Behälter (2) bereitgestellt wird, der eine flexible Wand (3) aufweist, die einen Innenraum (4) begrenzt, der mit einem biopharmazeutischen Fluid (C) befüllbar ist, wobei der Behälter (2) umfasst:
o eine Mischvorrichtung (7), die eine Welle (8) aufweist, die eine gemäß einer Hauptachse (XX) einstellbare Länge aufweist, wobei sich die Welle (8) gerade zwischen einem unteren Ende (8a) und einem oberen Ende (8b) erstreckt,
o ein erstes Lager (11), das an der Wand (3) befestigt ist, wobei sich die Welle (8) mindestens in den Innenraum (4) ab dem ersten Lager (11) erstreckt,
- eine äußere starre Aufnahmevorrichtung (18) des Behälters (2) bereitgestellt wird,
- ein Antriebsmotor (9) bereitgestellt wird, der sich außerhalb des Behälters (2) befindet, wobei der Motor (9) geeignet ist, die Welle (8) der Mischvorrichtung (7) rotatorisch anzutreiben,
- der Behälter (2) in der äußeren starren Aufnahmevorrichtung (18) platziert wird, wobei die äußere starre Aufnahmevorrichtung (18) eine Bodenwand (19) und eine Umfangswand (20) umfasst, die einen Aufnahme begrenzen, die geeignet ist, den Behälter (2) aufzunehmen, wobei die flexible Wand (3) des Behälters (2) auf der Bodenwand (19) der äußeren starren Aufnahmevorrichtung (18) angeordnet ist, und
- da die Position des Motors im Verhältnis zu der äußeren starren Aufnahmevorrichtung (18) fest ist und der Behälter (2) bereits in der äußeren starren Aufnahmevorrichtung (18) platziert ist, die Länge der Welle (8) gemäß der Hauptachse (XX) eingestellt wird, indem die Welle (8) dem Motor (9) zugewandt auf der Seite des oberen Endes (8b) angeordnet wird, um es dem Motor (9) zu gestatten, die Welle (8) rotatorisch anzutreiben, wobei sich die Welle (8) gemäß der Hauptachse (XX) vertikal erstreckt mit dem unteren Ende (8a), das sich in Richtung eines unteren Teils (3a) des Behälters (2) befindet, wohingegen sich das obere Ende (8b) in Richtung eines oberen Teils (3c) des Behälters (2) befindet, indem es mit dem ersten Lager (11) verbunden ist,
und wobei die Welle (8) einen ersten Teil (24) und einen zweiten Teil (25) aufweist, die gemäß der Hauptachse (XX) relativ zueinander translatorisch beweglich sind, wobei der erste Teil (24) ein Element (27) umfasst, das geeignet ist, in einem geraden Schlitz (28) des zweiten Teils (25) der Welle (8) zu gleiten, um die Länge der Welle (8) einzustellen, und wobei das erste Lager (11) in unmittelbarer Nähe des Motors (9) in einem Abstand vom Motor (9) platziert ist, der kleiner als die Länge (lf) des geraden Schlitzes (28) ist.

2. Verfahren nach Anspruch 1, wobei der Motor (9) geeignet ist, die Welle (8) durch magnetischen Rotationsantrieb der Welle (8) rotatorisch anzutreiben, wobei das erste Lager (11) einen starren Flansch (16) aufweist, der fest und dicht an einem oberen Teil (3c) der Wand (3) des Behälters (2) befestigt ist,
und wobei der obere Teil (3c) der flexiblen Wand (3) in den Bereich des Motors (9) geführt wird, damit eine antreibende Drehscheibe (30), die zum Motor (9) gehört und sich außerhalb des Behälters (2) befindet, in einem Hohlraum (16b) des Flanschs (16) angeordnet wird, wobei die antreibende Drehscheibe (30) funktionell mit einer angetriebenen Drehscheibe (15) zusammenwirkt, die an der Welle (8) am oberen Ende (8b) befestigt ist, wobei die angetriebene Drehscheibe (15) eine Vielzahl von Magneten (17) umfasst und imstande ist, im Verhältnis zum Flansch (16) zu drehen.

3. Verfahren nach Anspruch 1 oder 2, wobei sich der Behälter (2) in einem zerlegten Zustand ohne biopharmazeutisches Fluid (C) befindet, wenn er in der äußeren starren Aufnahmevorrichtung (18) platziert wird.

4. Verfahren nach Anspruch 1, 2 oder 3, wobei die Länge der Welle (8) auf einem Weg einstellbar ist, der im Wesentlichen dem axialen Platzbedarf des Motors (9) entspricht.

5. Verfahren nach einem der Ansprüche 1 bis 4, wobei sich die Welle (8) ganz im Innenraum (4) befindet und die Länge der Welle (8) gemäß der Hauptachse (XX) eingestellt wird, indem das erste Lager (11) dem Motor (9) zugewandt positioniert wird, um dem Motor (9) zu gestatten, die Welle (8) rotatorisch anzutreiben.

6. Verfahren nach einem der vorhergehenden Ansprüche, wobei der Behälter (2) ebenfalls ein zweites Lager (12) umfasst, das an der Wand (3) befestigt ist, und das zweite Lager (12) mit der äußeren starren Aufnahmevorrichtung (18) verbunden wird, nachdem der Behälter (2) über eine Zugangsöffnung (21) in der äußeren starren Aufnahmevorrichtung (18) platziert wurde, wobei die Zugangsöffnung (21) in der Umfangswand (20) vorgesehen ist und das Platzieren und das Herausnehmen des Behälters (2) gestattet.

7. Vorrichtung nach einem der vorhergehenden Ansprüche, wobei der Behälter (2), der mit einem Entleerungsport (6) versehen ist, lösbar in der äußeren starren Aufnahmevorrichtung (18) platziert ist, wobei der Behälter (2) zur einmaligen Verwendung ist.

8. Mischbehälter (1), der bestimmt ist, durch das Verfahren zum Zusammenbauen nach einem der vorhergehenden Ansprüche zusammengebaut zu werden, umfassend:
- den Behälter (2), dessen von einer flexiblen Wand (3) begrenzter Innenraum (4) imstande ist, mit einem biopharmazeutischen Fluid (C) gefüllt zu werden, wobei der Behälter (2) umfasst:
o die Mischvorrichtung (7), die die Welle (8) aufweist, die sich gerade erstreckt und deren Länge gemäß einer Hauptachse (XX) einstellbar ist,
o das erste Lager (11), ab dem sich die Welle (8) in den Innenraum (4) erstreckt,
- den Antriebsmotor (9), der sich außerhalb des Behälters (2) befindet, wobei der Motor (9) geeignet ist, die Welle (8) der Mischvorrichtung (7) rotatorisch anzutreiben, und
- die äußere starre Aufnahmevorrichtung (18), deren Bodenwand (19) und Umfangswand (20) die Aufnahme begrenzen, die geeignet ist, den Behälter (2) aufzunehmen,
**dadurch gekennzeichnet, dass** der Motor (9() im Verhältnis zu der äußeren starren Aufnahmevorrichtung (18) fest ist, wobei die Länge der Welle (8) gemäß der Hauptachse (XX) einstellbar ist, damit sich die Welle (8) vertikal gemäß der Hauptachse (XX) erstreckt, indem sie das untere Ende (8a) präsentiert, das sich in Richtung eines unteren Teils (3a) des Behälters (2) befindet und das obere Ende (8b), das sich in Richtung eines oberen Teils (3c) des Behälters (2) befindet, indem es mit dem ersten Lager (11) verbunden ist,
wobei sich die Welle (8) mit einstellbarer Länge zwischen dem ersten Lager (11) und einem zweiten Lager (12) erstreckt, wobei jedes von dem ersten Lager (11) und dem zweiten Lager (12) an der flexiblen Wand (3) des Behälters (2) befestigt sind,
und wobei die Welle (8) einen ersten Teil (24) und einen zweiten Teil (25) umfasst, die gemäß der Hauptachse (XX) relativ zueinander translatorisch bewegbar sind, wobei der erste Teil ein Element (27) umfasst, das geeignet ist, in einem geraden Schlitz (28) des zweiten Teils (25) der Welle (8) zu gleiten.

9. Mischbehälter (1) nach Anspruch 8, wobei der Motor (9) geeignet ist, den magnetischen Antrieb der Welle (8) zu gestatten und eine antreibende Drehscheibe (30) umfasst, die sich außerhalb des Behälters (2) befindet, wobei die antreibende Drehscheibe (30) funktionell mit einer angetriebenen Drehscheibe (15) zusammenwirkt, die an der Welle (8) befestigt ist.

10. Mischbehälter (1) nach Anspruch 9, wobei das erste Lager (11) einen starren Flansch (16) aufweist, der fest und dicht an einem oberen Teil (3c) der Wand (3) des Behälters (2) befestigt ist, wobei der Flansch (16) einen äußeren Ringbund (16a) umfasst, mit dem der Motor (9) mit dem Behälter (2) verbunden ist, und wobei der Flansch (16) mit der Welle (8) im Innenraum (4) des Behälters (2) über die angetriebene Drehscheibe (15) verbunden ist, wobei die angetriebene Drehscheibe (15) relativ frei im Verhältnis zum Flansche (16) drehen kann.

11. Mischbehälter (1) nach einem der Ansprüche 8 bis 10, wobei die Welle (8) mindestens ein Mischorgan (10) trägt und antreibt, das geeignet ist, das in dem Innenraum (4) befindliche biopharmazeutische Fluid (C) zu mischen.

## Claims

1. A method for assembling a mixer-container (1) intended to receive a biopharmaceutical fluid (C) in order to mix it, wherein:
- a container (2) is provided, having a flexible wall (3) defining an inner space (4) suitable for filling with biopharmaceutical fluid (C), the container (2) comprising:
o a mixing device (7) having a shaft (8) having an adjustable length along a main axis (XX), the shaft (8) extending rectilinearly between a lower end (8a) and an upper end (8b),
o a first bearing (11) attached to the wall (3), the shaft (8) extending at least into the inner space (4) from the first bearing (11),
- a rigid outer containment device (18) of the container (2) is provided,
- a drive motor (9) is provided, located outside the container (2), the motor (9) being suitable for rotating the shaft (8) of the mixing device (7),
- the container (2) is placed in the rigid outer containment device (18), the rigid outer containment device (18) comprising a bottom wall (19) and a peripheral wall (20) defining a housing suitable for receiving the container (2), the flexible wall (3) of the container (2) being arranged on the bottom wall (19) of the rigid outer containment device (18), and
- knowing that the position of the motor is fixed relative to the rigid outer containment device (18) and that the container (2) is already placed in the rigid outer containment device (18), the length of the shaft (8) along the main axis (XX) is adjusted by arranging the shaft (8) facing the motor (9), on the side of the upper end (8b), in order to enable the motor (9) to rotate the shaft (8), the shaft (8) extending vertically along the main axis (XX) with the lower end (8a) located towards a lower part (3a) of the container (2) while the upper end (8b) is located towards an upper part (3c) of the container (2), being connected to the first bearing (11),
and wherein the shaft (8) has a first part (24) and a second part (25) which are movable in translation relative to one another along the main axis (XX), the first part (24) comprising an element (27) suitable for sliding in a rectilinear slot (28) of the second part (25) of the shaft by adjusting the length of the shaft (8), and wherein the first bearing (11) is placed in the immediate vicinity of the motor (9), at a distance from the motor (9) less than the length (If) of the rectilinear slot (28).

2. The method according to claim 1, wherein the motor (9) is suitable for rotating the shaft (8), by magnetic rotating of said shaft (8), the first bearing (11) having a rigid flange (16) rigidly and sealingly fixed to an upper part (3c) of the wall (3) of the container (2),
and wherein the upper part (3c) of the flexible wall (3) is brought to the motor (9) in order that a rotary drive disc (30) belonging to the motor (9) and located outside the container (2) is arranged in a cavity (16b) of the flange (16), the rotary drive disc (30) operatively engaging with a rotary driven disc (15) attached to the shaft (8) at the upper end (8b), the rotary driven disc (15) comprising a plurality of magnets (17) and being suitable for turning relative to the flange (16).

3. The method according to claim 1 or 2, wherein the container (2) is in a disassembled state, empty of biopharmaceutical fluid (C), when it is placed in the rigid outer containment device (18).

4. The method according to claim 1, 2 or 3, wherein the length of the shaft (8) is adjustable on a stroke substantially corresponding to the axial size of the motor (9).

5. The method according to any one of claims 1 to 4, wherein the shaft (8) is entirely located in the inner space (4) and the length of the shaft (8) along the main axis (XX) is adjusted by positioning the first bearing (11) facing the motor (9) in order to allow the motor (9) to rotate the shaft (8).

6. The method according to any one of the preceding claims, wherein the container (2) also comprises a second bearing (12) attached to the wall (3) and the second bearing (12) is connected to the rigid outer containment device (18) after having placed, via an access opening (21), the container (2) in the rigid outer containment device (18), the access opening (21) being provided in the peripheral wall (20) and enabling the placing and removal of the container (2).

7. The method according to any one of the preceding claims, wherein the container (2) provided with a drainage port (6) is removably placed in the rigid outer containment device (18), said container (2) being disposable.

8. A container-mixer (1) intended to be assembled by the assembly method according to any one of the preceding claims, comprising:
- said container (2) for which the inner space (4) defined by the flexible wall (3) is suitable for being filled with biopharmaceutical fluid (C), the container (2) comprising:
o the mixing device (7) having the shaft (8) which extends rectilinearly and the length of which is adjustable along a main axis (XX),
o the first bearing (11) from which the shaft (8) extends into the inner space (4),
- the drive motor (9) located outside the container (2), the motor (9) being suitable for rotating the shaft (8) of the mixing device (7), and
- the rigid outer containment device (18), the bottom wall (19) and the peripheral wall (20) of which define the housing suitable for receiving the container (2),
**characterised in that** the motor (9) is fixed relative to the rigid outer containment device (18), the length of the shaft (8) being adjustable along the main axis (XX) in order that the shaft (8) extends vertically along the main axis (XX) having said lower end (8a) located towards a lower part (3a) of the container (2) and said upper end (8b) located towards the upper part (3c) of the container (2), being connected to the first bearing (11),
wherein the shaft (8) of adjustable length extends between the first bearing (11) and a second bearing (12), each of the first bearing (11) and second bearing (12) being fixed to the flexible wall (3) of said container (2),
and wherein the shaft (8) comprises a first part (24) and a second part (25), movable in translation relative to one another along the main axis (XX), the first part comprising an element (27) suitable for sliding in a rectilinear slot (28) of the second part (25) of the shaft (8).

9. The container-mixer (1) according to claim 8, wherein the motor (9) is suitable for enabling the magnetic driving of the shaft (8) and comprises a rotary drive disc (30) located outside of the container (2), the rotary drive disc (30) operatively engaging with a rotary driven disc (15) attached to the shaft (8).

10. The container-mixer (1) according to claim 9, wherein the first bearing (11) has a rigid flange (16) rigidly and sealingly fixed to an upper part (3c) of the wall (3) of the container (2), the flange (16) comprising an outer annular collar (16a) with which the motor (9) is connected to the container (2), and wherein the flange (16) is connected to the shaft (8) in the inner space (4) of the container (2) by means of the rotary driven disc (15), the rotary driven disc (15) being able to turn relatively freely relative to the flange (16).

11. The container-mixer (1) according to any one of claims 8 to 10, wherein the shaft (8) supports and drives at least one mixing member (10) suitable for mixing the biopharmaceutical fluid (C) located in the inner space (4).
